# EUROPÄISCHE PATENTSCHRIFT

(11) **EP 1 928 477 B1**
(45) Veröffentlichungstag und Bekanntmachung des Hinweises auf die Patenterteilung: **14.08.2013**
(21) Anmeldenummer: 06791962.1
(22) Anmeldetag: 11.09.2006
(51) Int. Cl.: A61K 33/38, A61K 45/06

(54) **ANTIMIKROBIELLES MEDIZINTECHNISCHES PRODUKT, VERFAHREN ZU SEINER HERSTELLUNG UND VERWENDUNG**
ANTIMICROBIAL MEDICAL PRODUCT METHOD FOR PRODUCTION AND USE THEREOF
PRODUIT MEDICAL ANTIMICROBIEN, PROCEDE DE FABRICATION ET UTILISATION

(30) Priorität: 09.09.2005 DE 102005044361
(43) Veröffentlichungstag der Anmeldung: 11.06.2008
(73) Patentinhaber: Aesculap AG, 78532 Tuttlingen (DE)
(72) Erfinder: ODERMATT, Erich, K., CH- Schaffhausen (CH); BARGON, Rainer, 88512 Mengen (DE)
(74) Vertreter: Patentanwälte Ruff, Wilhelm, Beier, Dauster & Partner
(86) Internationale Anmeldenummer: PCT/EP2006/008813
(87) Internationale Veröffentlichungsnummer: WO 2007/028645

(56) Entgegenhaltungen:
- WO-A2-2004/056404
- CA-A1- 2 529 236
- DE-A1- 10 323 597
- US-A1- 2002 022 012
- PARK ET AL: "Effects of silver nanoparticles on the fluidity of bilayer in phospholipid liposome" COLLOIDS AND SURFACES. B, BIOINTERFACES, ELSEVIER, AMSTERDAM,, NL, Bd. 44, Nr. 2-3, August 2005 (2005-08), Seiten 117-122, XP005006184 ISSN: 0927-7765

## Beschreibung

Die Erfindung betrifft ein medizintechnisches Produkt mit einer antimikrobiellen Ausstattung, ein Verfahren zu dessen Herstellung sowie die Verwendung der antimikrobiellen Ausstattung als Biozid bei medizintechnischen Produkten.

Die Prävention von Keim- bzw. Schadorganismenbesiedlung von Oberflächen ist insbesondere im medizinischen Bereich von überragender Bedeutung. So sind beispielsweise nach einem operativen Eingriff traumatisierte Körperstellen bzw. Wundoberflächen für die Keimvermehrung besonders anfällig. Infektionen mit endogenen Keimen, beispielsweise pathogenen Escherichia coli oder Staphylococcus aureus Erregern sind bei vielen Patienten unauffällig und werden daher vor Operationsbeginn häufig übersehen. Prinzipiell besteht innerhalb der ersten 24 Stunden nach der Operation ein Risiko durch eine äußere sogenannte nosokomiale Infektion der Wundstelle. Dieses Risiko kann insbesondere durch eine falsche Naht und/oder Knotentechnik und eine damit einhergehende Minderdurchblutung der betroffenen Körperstelle bzw. des betroffenen Gewebes erhöht werden. Bei stark abwehrgeschwächten Patienten besteht zusätzlich ein postoperatives Risiko durch die interne Verschleppung körpereigener Keime, die in einer veränderten Umgebung, beispielsweise im Wundmilieu, durch Ausbildung sogenannter Virulenzfaktoren zu pathogenen Erregern konvertieren können. Es besteht daher auch ein großes Interesse, medizintechnische Produkte während und nach dem operativen Eingriff keimfrei zu halten. Da eine anfängliche Sterilität dieser Produkte eine spätere Keimbesiedlung auf der Oberfläche nicht verhindern kann, wurden zunehmend Produkte hergesellt, die antimikrobiell bzw. biozid ausgestattet bzw. ausgerüstet sind. Zahlreiche Übergangsmetalle, vor allem Silber, sind dafür bekannt, in ihrer elementaren und/oder ionogenen Form antimikrobiell zu wirken. So ist beispielsweise bekannt, dass Silber eine sehr starke Wechselwirkung mit thiolisierten Verbindungen in Bakterienzellen aufweist. Durch diese Wechselwirkung werden insbesondere Sauerstoff- und Stoffwechsel-Enzyme, die bei einzelligen Mikroorganismen, insbesondere Keimen, beispielsweise Bakterien und Pilzen, für die Sauerstoffgewinnung verantwortlich sind, deaktiviert. Die Atmungskette der Mikroorganismen wird somit unterbrochen, wodurch die Mikroorganismen in kürzester Zeit "ersticken".

Daher wurden zahlreiche Zusammensetzungen auf der Basis von Silber bzw. Silberionen zur antimikrobiellen Ausrüstung von insbesondere Implantaten entwickelt. So wird beispielsweise in der WO 8102667 die antimikrobiell Oberflächenbeschichtung von endoprosthetischen Implantaten und Nahtmaterialien mit einer bioabbaubaren metallischen Silberlegierung beschrieben, wobei eine Freisetzung von Silberionen in vivo beim Abbau der Legierung erfolgt. In der WO 04/054503 bzw. WO 04/052314 werden antimikrobielle Zusammensetzungen aus Metallsalzen von Fettsäuren bzw. Fettsäureestern beschrieben. Die Verwendung von Silbersalzen führt jedoch in manchen Fällen zu einer lokale Anhäufung von Silberionen im menschlichen oder tierischen Organismus, wodurch in manchen Fällen unerwünschte, insbesondere allergische oder (gewebe-)toxische, Nebenwirkungen auftreten können.

DE10323597 beschreibt ein medizintechnisches Produkt welches einen Hybridkomplex bestehend aus einem verzweigten amphiphilen Makromolekül und einen Metallnanopartikel beinhaltet.

WO2004056404 beschreibt die Verwendung eines Überzugs bestehend aus Metallnanopartikeln und einem polyionischen Material für medizinische Geräte. US20020022012 beschreibt ein Dendrimer-Silber-Nanokomposit als Bakterizid.

Aufgabe der vorliegenden Erfindung ist es daher, medizintechnische Produkte bereitzustellen, die in einer besonders schonenden und körperverträglichen Weise antimikrobiell bzw. biozid wirken und gleichzeitig bezüglich ihrer antimikrobiellen Eigenschaften äußerst wirksam sind.

Diese Aufgabe wird gelöst durch ein medizintechnisches Produkt mit einer antimikrobiellen bzw. bioziden Ausstattung aus einem Komplexmaterial aus Metallnanopartikeln und Liganden, wobei es sich bei den Liganden zur Verhinderung einer Agglomeration der Partikel um eine oberflächenaktive Substanz handelt.

Unter einer antimikrobiellen bzw. bioziden Ausstattung im Sinne der vorliegenden Erfindung soll ein Komplexmaterial verstanden werden, das Zellwachstum bzw. -proliferation von Mikroorganismen, insbesondere von Keimen, verhindert und/oder die Abtötung von vorhandenen Mikroorganismenkolonien, insbesondere Keimkolonien, bewirkt.

Vorzugsweise ist die antimikrobielle Ausstattung auf der Oberfläche des Produktes, insbesondere in Form einer Beschichtung, vorgesehen, wobei die Ausstattung zur Oberfläche des Produktmaterials durch geeignete Wechselwirkungen, insbesondere elektrostatische Anziehungskräfte, Wasserstoffbrückenbindungen und/oder Van-der-Waals-Interaktionen, eine hinreichend stabile Haftverbindung ausbildet. Vorzugsweise liegt die antimikrobielle Ausstattung auf der Oberfläche des Produktes, insbesondere in Form einer Beschichtung, vor. Auf diese Weise wird beispielsweise ein Ablösen, insbesondere Abwischen oder Abwaschen, der bioziden Ausstattung vom beschichteten Produkt verhindert und eine wirkungsvolle Prävention des derart ausgestatteten Produktes gegen mikrobielle Besiedelung und Proliferation nach Einführung in den Körper und/oder nach einer mehr oder weniger langen postoperativen Verweildauer im Körper garantiert.

Zusätzlich oder alternativ zu der soeben erwähnten Ausführungsform kann es erfindungsgemäß vorteilhaft sein, dass sich das Komplexmaterial innerhalb des Produktes befindet. Dies kann besonders bevorzugt sein, wenn es sich bei dem Werkstoff um ein Polymer oder ein anderes Material handelt, dessen Herstellungsprozess die Einführung des Komplexmaterials in das Innere des Produktes erlaubt. Auf diese Weise kann insbesondere eine verzögerte und gleichmäßige Freisetzungsrate des bioziden Komplexmaterials von allen Seiten des Produktes an die Umgebung, insbesondere an das umliegende Gewebe, erreicht werden.

In einer weiteren Ausführungsform wird jeder Metallnanopartikel von mindestens einer oberflächenaktiven Substanz umgeben, wobei die oberflächenaktive Substanz den Metallnanopartikel vorzugsweise von allen Seiten hüllenartig einschließt. Bevorzugt ist der polare, insbesondere geladene, Teil der oberflächenaktiven Substanz zum Metallnanopartikel hin orientiert und ermöglicht durch die im polaren Teil befindlichen Heteroatome, beispielsweise Stickstoff- und/oder Sauerstoffatome, bzw. Heteroatomgruppierungen eine koordinative Bindung zu dem Metallnanopartikel. Erfindungsgemäß kann der Metallnanopartikel auf diese Weise partiell positiv polarisiert sein.

Gemäß der Erfindung handelt es sich bei der oberflächenaktiven Substanz um mindestens einen Vertreter aus der Gruppe von Alkylammoniumsalze, um ein sogenanntes quartäres Alkylammoniumsalz, insbesondere um ein Cetyltrimethylammoniumhalogenid. Cetyltrimethylammoniumbromid (CTAB) ist wegen seiner außerordentlichen antimikrobiellen bzw. bioziden Eigenschaften besonders bevorzugt.

Mit Vorteil handelt es sich bei den Metallnanopartikeln um Silber- oder Kupfernanopartikel, wobei Silbernanopartikel besonders bevorzugt sind. Bei den Metallnanopartikeln handelt es sich nicht um ionische sondern um elementare metallische Teilchen. Silber und in geringerem Umfang auch Kupfer stellen hinsichtlich den zu bekämpfenden Mikroorganismen, insbesondere Staphylokokken, beispielsweise Staphylococcus aureus, und Enterobakterien, beispielsweise Escherichia coli und Pseudomonas aeruginosa, die am stärksten toxischen Metalle dar.

Vorzugsweise weisen die Metallnanopartikel eine zerklüftete Morphologie auf, wodurch sich ein besonders großes Oberflächen/VolumenVerhältnis ergibt.

In einer weiteren Ausführungsform weisen die Metallnanopartikel einen Durchmesser von 80 bis 180 nm, insbesondere von 100 bis 150 nm, auf. Der verhältnismäßig kleine Durchmesser der Metallnanopartikel ist das Ergebnis einer äußerst wirksamen gegenseitigen Abschirmung durch die daran koordinativ gebundene oberflächenaktive Substanz. Auf diese Weise wird eine Agglomeration der Partikel zu größeren Partikelverbänden bzw. Agglomeraten und damit eine Verkleinerung des Oberflächen/Volumen-Verhältnisses der Partikel vermieden. Eine wirksame Abschirmung der Metallnanopartikel verhindert außerdem eine Anhäufung und/oder Ausfällung der Partikel im Körper, die schwerwiegende Nebenwirkungen, insbesondere Gewebeschädigungen, verursachen können.

Das erfindungsgemäße Produkt weist vorzugsweise eine Beschichtung der Metallnanopartikel zwischen 0,05 und 3 Gew.-%, vorzugsweise zwischen 0,05 und 1 Gew.-%, bezogen auf das Gesamtgewicht des Produktes, auf.

Gemäß einer weiteren bevorzugten Ausführungsform weist das Produkt ein von der antimikrobiellen Ausstattung verschiedenes Material, insbesondere in Form einer Beschichtung, auf. Vorzugsweise ist das Material bioresorbierbar. Somit kann der Zeitpunkt, zu welchem die Umgebung, insbesondere Körperflüssigkeiten, mit der antimikrobiellen Ausstattung des Produktes in Kontakt treten, durch die Wahl des bioresorbierbaren Materials gezielt gesteuert werden. Bevorzugt handelt es sich bei dem bioresorbierbaren Material um ein Polymer, insbesondere um ein hydrolysierbares Polymer, vorzugsweise um ein Polyester, Polyurethan und/oder Silikon. Bei dem Polyester kann es sich insbesondere um einen aliphatischen Polyester handeln. Weiterhin kann es bevorzugt sein, dass es sich bei dem Polymer um ein Co- oder Terpolymer, insbesondere auf der Basis von Glykolsäure, ε-Caprolacton und/oder Trimethylencarbonat, handelt. Bei dem Polymer kann es sich insbesondere um ein Copolymer aus Trimethylencarbonat und ε-Caprolacton oder um ein Terpolymer aus Glykolsäure, ε-Caprolacton und Trimethylencarbonat handeln. Mit Vorteil beträgt der Polymergehalt am Gesamtgewicht des Produktes 1 bis 10 Gew.-%, vorzugsweise 1 bis 6 Gew.-%.

Bei dem medizintechnischen Produkt kann es sich erfindungsgemäß um ein eindimensionales Textilprodukt, insbesondere um eine Einzelfaser, handeln. Das erfindungsgemäße Produkt kann ein Mono- oder Multifilament sein. Bevorzugt handelt es sich bei dem erfindungsgemäßen Produkt um ein Garn.

In einer weiteren Ausführungsform handelt es sich bei dem erfindungsgemäßen Produkt um ein textiles Flächengebilde, insbesondere um ein Vlies, Gewebe, Gewirk, Geflecht oder Gestrick.

In einer weiteren Ausführungsform handelt es sich bei dem erfindungsgemäßen Produkt um ein temporäres oder dauerhaftes Implantat für den menschlichen oder tierischen Körper. Hierbei handelt es sich bei den mit der antimikrobiellen Ausstattung versehenen Implantaten vorzugsweise um Gelenkimplantate, Stents, Schrauben, Nägel und Platten. Die Implantate können aus Metallen und/oder Kunststoffen gebildet sein. Die Implantate können zum Beispiel zur Reparatur von Knochen- und/oder Knorpeldefekten verwendet werden. Weiterhin kann es sich bei den Implantaten um Herniennetze, Inkontinenzbänder, Gefäßprothesen, Membranen sowie um Folien, beispielsweise zur Adhäsionsprophylaxe, handeln. Weiterhin kommen als Implantate allgemein textile Implantate in Betracht. Durch die biozide Ausstattung dieser Implantate ist es möglich, diese in akut infizierte oder infektionsgefährdete Körperregionen einzuführen.

In einer anderen Ausführungsform handelt es sich bei den medizintechnischen Produkten um medizinische Instrumente, insbesondere um chirurgische Scheren, Zangen und Klammern sowie um Katheter oder Sonden und weitere Instrumente, insbesondere für minimalinvasive Eingriffe. In diesem Zusammenhang ist die wirkungsvolle Haftverbindung der antimikrobiellen Ausstattung mit der Oberfläche des erfindungsgemäßen Produktes von besonderem Vorteil, da die soeben bezeichneten medizinischen Instrumente einer besonders hohen mechanischen Beanspruchung, insbesondere durch Reiben und Wischen, ausgesetzt sind. Die Haftung der antimikrobiellen Ausstattung mit der Produktoberfläche wird insbesondere durch die lipophilen Wechselwirkungen, vorzugsweise Van-der-Waals-Wechselwirkungen, der insbesondere von den Metallnanopartikeln wegweisenden langkettigen aliphatischen und/oder aromatischen Resten der oberflächenaktiven Liganden mit der Oberfläche der Produktmaterialien bewirkt.

Bei den medizintechnischen Produkten kann es sich ferner um Erzeugnisse wie beispielsweise Drainageschläuche, Nahtmaterialien oder Wundauflagen handeln, wobei Nahtmaterialien bevorzugt sind.

Das medizintechnische Produkt besteht in einer bevorzugten Ausführungsform aus einem Metall oder einer Metalllegierung, insbesondere aus Titan, Edelstahl, Magnesium, Tantal oder einer Legierung davon. Medizintechnische Produkte aus Magnesium oder Tantal sind wegen ihrer Bioverträglichkeit und -resorbierbarkeit besonders bevorzugt.

In einer weiteren Ausführungsform handelt es sich bei dem erfindungsgemäßen Produkt um ein nicht resorbierbares oder mindestens teilweise resorbierbares Polymer. So kann es sich bei dem nicht resorbierbaren Polymer um ein Polyolefin, insbesondere Polyethylen und/oder Polypropylen, handeln. Erfindungsgemäß kann es vorgesehen sein, dass es sich bei dem polymeren bioresorbierbaren Material um ein hydrolysierbares Polymer, vorzugsweise ein Polyester, Polyurethan und/oder Silikon, handelt.

In einer weiteren Ausführungsform kann es sich bei dem Material des medizintechnischen Produktes auch um einen keramischen Werkstoff handeln. Mit Vorteil kann es sich um einen resorbierbaren keramischen Werkstoff, insbesondere um Hydroxylapatit oder Tricalciumphosphat, handeln.

Gemäß einer weiteren Ausführungsform weist das Produkt Poren, vorzugsweise interkonnektierende Poren, auf. Dies kann besonders vorteilhaft sein, da auf diese Weise eine vergrößerte Oberfläche für die antimikrobielle Ausstattung zur Verfügung steht. Somit kann eine größere Menge des bioziden Komplexmaterials auf das auszustattende Produkt aufgebracht werden. Im Falle des Vorliegens eines interkonnektierenden Porensystems kann außerdem eine gewünschte Menge des antimikrobiellen Komplexmaterials auch innerhalb des auszustattenden Produktes eingebracht sein.

Das Produkt ist mit Vorteil sterilisierbar und liegt insbesondere in sterilisierter Form vor. Als Sterilisierungsmethoden kommen alle dem Fachmann bekannten Methoden, insbesondere Bestrahlung, Dampfsterilisation oder Ethylenoxidbegasung, in Frage, die vorzugsweise die chemische Struktur und/oder die antimikrobiellen Eigenschaften des Komplexmaterials nicht beeinträchtigen. Das erfindungsgemäße medizintechnische Produkt liegt im Gebrauchszustand vorzugsweise in steriler Form vor.

Gegenstand der Erfindung ist weiterhin ein Verfahren zur Herstellung eines medizintechnischen Produktes, wobei ein Komplexmaterial aus Metallnanopartikeln und einer oberflächenaktiven Substanz, insbesondere in Form einer Lösung, von außen auf das nicht ausgestattete Produkt aufgebracht wird. Die Aufbringung des Komplexmaterials kann insbesondere durch Pressen, Walzen, Rakeln, Sprühen oder Tauchen erfolgen. Vorzugsweise wird das antimikrobielle Komplexmaterial im Tauchverfahren auf die Oberfläche des nicht ausgestatteten Produktes aufgebracht. Dies kann besonders vorteilhaft für die Herstellung von antimikrobiell ausgestatteten Netzen oder Bändern, insbesondere Harninkontinenzbändern, sein. Ebenso ist es möglich, das biozide Komplexmaterial im Durchzugsverfahren von außen auf das nicht ausgestattete Produkt aufzubringen, insbesondere auf Nahtmaterialien, Netze und Bänder. Bei der Lösung handelt es sich bevorzugt um ein sogenanntes Organosol des betreffenden Metalls, insbesondere von Silber oder Kupfer, welches das Metall in Form von Nanopartikeln insbesondere in einer kolloidalen Verteilung in einem organischen Lösungsmittel enthält. Die Gewinnung eines solchen Metallorganosols erfolgt vorzugsweise ausgehend von einer wässrigen Metallsalzlösung, wobei das oxidierte Metall in Gegenwart eines geeigneten Reduktionsmittels, insbesondere von Natriumborhydrid, Vitamin C oder einem Aldehyd, zu elementarem Metall in Form von Nanopartikeln reduziert wird. Aus dem so hergestellten Metallhydrosol werden die darin kolloidal verteilten Metallnanopartikel beispielsweise durch Zusatz von Phasentransferinduktoren bzw. - katalysatoren in ein organisches Lösungsmittel extrahiert. Bei dem organischen Lösungsmittel kann es sich um einen Alkohol, beispielsweise um Isopropanol oder Propanol, einen aliphatischen Ester, beispielsweise Ethyl- oder Butylacetat, oder ein aromatisches Lösungsmittel, beispielsweise Toluol oder Xylol, handeln. Im Falle eines in Wasser mindestens teilweise löslichen bzw. mischbaren Lösungsmittels kann die Zugabe eines Additivs, beispielsweise eines Salzes, zur Erzeugung und/oder Verbesserung einer Phasengrenze vorteilhaft sein. Die Metallgehalte der auf diese Weise erhaltenen Lösungen, insbesondere Organosole, liegen üblicherweise zwischen 0,05 und 10 Gew.-%, insbesondere zwischen 0,05 und 5 Gew.-%. Vorzugsweise weisen die organischen Lösungen, insbesondere Organosole, einen Silbergehalt zwischen 0,05 und 10 Gew.-%, insbesondere zwischen 0,05 und 5 Gew.-%, auf.

Außerdem betrifft die Erfindung ein Verfahren zur Herstellung eines medizintechnischen Produktes, wobei ein antimikrobielles Komplexmaterial aus Metallnanopartikeln und einer oberflächenaktiven Substanz, insbesondere in Form einer Lösung, in den Werkstoff des Produktes bei dessen Herstellung zugegeben wird. Vorzugsweise handelt es sich bei der Lösung um ein Organosol des betreffenden kolloidal verteilten Metalls, insbesondere von Silber oder Kupfer, in Nanopartikelform. Durch die Zugabe zum Werkstoff des Produktes wird eine gleichmäßige Verteilung des antimikrobiellen Komplexmaterials innerhalb des medizintechnischen Produktes erreicht. Bezüglich weiterer Einzelheiten und Merkmale wird auf die obige Beschreibung verwiesen.

Gemäß einer weiteren Ausführungsform wird zusätzlich ein resorbierbares Polymer, insbesondere in Form einer Lösung, auf die Oberfläche des Produktes aufgebracht oder zu dem Werkstoff des Produktes bei dessen Herstellung zugegeben. So kann es bevorzugt sein, dass das erfindungsgemäße Produkt nach einer oberflächlichen Beschichtung mit dem antimikrobiellen Komplexmaterial in einem zweiten Beschichtungsverfahren mit einer zweiten Schicht eines resorbierbaren Polymers, insbesondere eines Polyesters, Polyurethans oder Silikons, versehen wird. Vorzugsweise wird als zweite Schicht ein resorbierbares Copolymer, insbesondere aus Trimethylencarbonat und ε-Caprolacton, oder ein resorbierbares Terpolymer, insbesondere aus Glykolsäure, ε-Caprolacton und Trimethylencarbonat, aufgebracht. Als Lösungsmittel können Alkohole, aliphatische Ester oder aromatische. Lösungsmittel eingesetzt werden, wobei Ethylacetat besonders bevorzugt ist. Vorzugsweise beträgt der Polymergehalt am medizintechnischen Produkt 1 bis 10 Gew.-%, vorzugsweise 1 bis 6 Gew.-%.

Alternativ dazu kann das resorbierbare Polymer und das antimikrobielle Komplexmaterial gemeinsam in einem Beschichtungsprozess auf das medizintechnische Produkt aufgebracht werden. Dies ist besonders vorteilhaft, da ein einziger Beschichtungsprozess wirtschaftlicher und damit kostengünstiger ist.

In einer weiteren vorteilhaften Ausführungsform wird das antimikrobielle Komplexmaterial mit dem Produktwerkstoff gemischt und anschließend zum gewünschten Produkt geformt, insbesondere extrudiert, gesponnen, gepresst, gewalzt, gegossen oder geblasen. Besonders bevorzugt wird eine Mischung aus dem antimikrobiellen Komplexmaterial und einem Polymer zu einem Fadenmaterial versponnen, welches je nach Art des verwendeten Polymers entweder zu resorbierbaren oder zu nicht resorbierbaren Nahtmaterialien oder zu textilen Produkten verarbeitet, insbesondere verwebt oder verwirkt, werden kann.

In einer anderen Ausführungsform des erfindungsgemäßen Produktes wird das antimikrobielle Komplexmaterial und ein bioresorbierbares Polymer mit dem Produktwerkstoff gemischt und anschließend zum gewünschten Produkt geformt, insbesondere extrudiert, gesponnen, gepresst, gewalzt, gegossen oder geblasen. Bezüglich weiterer Einzelheiten und Merkmale wird auf das bisher Gesagte Bezug genommen.

Weiterhin umfasst die Erfindung die Verwendung eines Komplexmaterials aus Metallnanopartikeln und einer oberflächenaktiven Substanz, wobei insbesondere jeder Metallnanopartikel hüllenartig von mindestens einer oberflächenaktiven Substanz umgeben ist, als Biozid bei einem medizintechnischen Produkt.

Das erfindungsgemäße Produkt verfügt durch die Art seiner Ausstattung über äußerst wirkungsvolle antimikrobielle bzw. biozide Eigenschaften, die insbesondere eine Langzeitwirkung aufweisen. Durch die Nanostruktur der Metallpartikel wird eine große Wirkstoffoberfläche bereitgestellt, die eine ausreichende Freisetzung von antimikrobiell wirksamen Metallionen, insbesondere Silberionen, ermöglicht. Gleichzeitig bewirkt die Unlöslichkeit des elementaren Metalls im wässrigen physiologischen Milieu eine sehr langsame und damit gewebeverträgliche Freisetzung der antimikrobiell wirksamen Metallionen. Durch die Kombination mit oberflächenaktiven, insbesondere im menschlichen und/oder tierischen Körper vorkommenden, Liganden oder Derivaten davon kann die Gewebeverträglichkeit eines derart ausgestatteten medizintechnischen Produktes zusätzlich erhöht werden. Weiterhin kann durch die Anwesenheit eines zusätzlichen resorbierbaren Polymers auf der Oberfläche und/oder innerhalb des Produktes der Zeitpunkt gezielt gesteuert werden, an welchem die Umgebung, insbesondere Körperflüssigkeiten, in Kontakt mit der antimikrobiellen Ausstattung des Produktes gelangen.

Weitere Einzelheiten und Merkmale der Erfindung ergeben sich aus der nachfolgenden Beschreibung bevorzugter Ausführungsformen anhand der Figuren, des Beispiels sowie der Unteransprüche. In diesen Ausführungsformen können einzelne Merkmale der Erfindung allein oder zu mehreren in Kombination mit anderen verwirklicht sein. Sämtliche Figuren werden hiermit durch ausdrückliche Bezugnahme zum Inhalt dieser Beschreibung gemacht.

In den Figuren ist folgendes gezeigt:

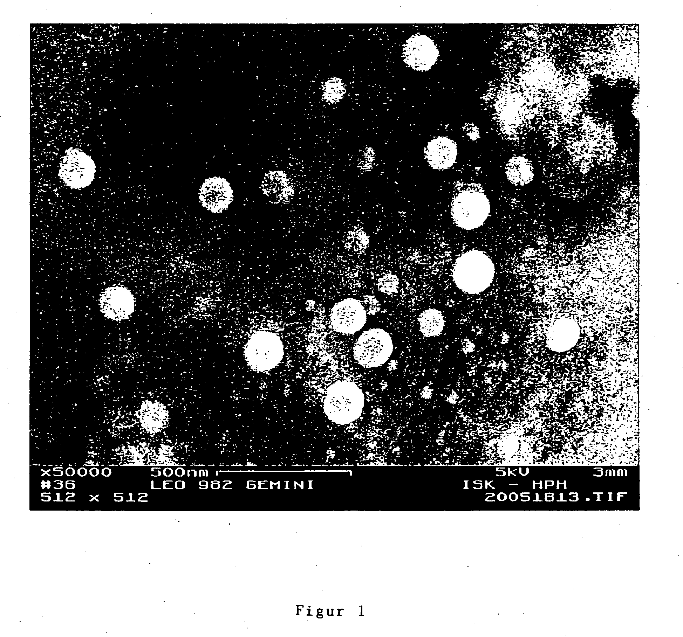

- Fig. 1:: Rasterelektronenmikroskopaufnahme eines Polyglykolsäurefadens der Stärke USP 2-0 mit einer Ag°/CTAB-Beschichtung (50.000fache Vergrößerung),

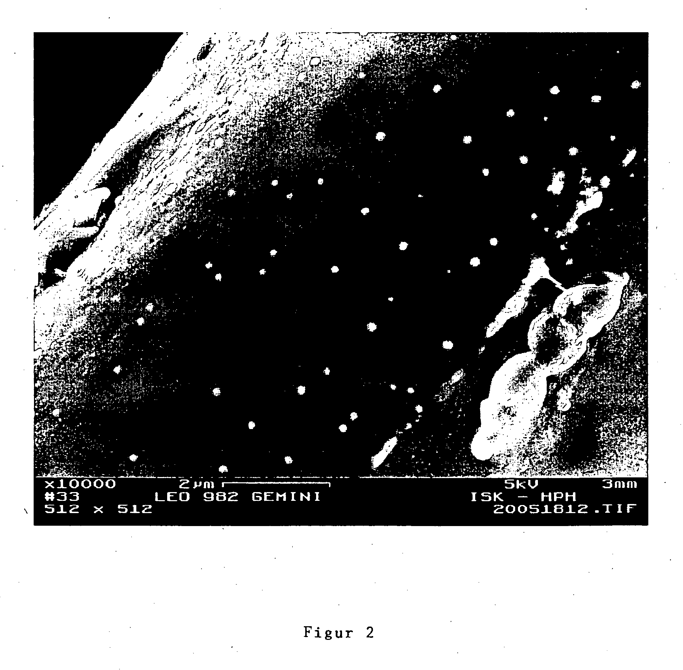

- Fig. 2:: Rasterelektronenmikroskopaufnahme eines Polyglykolsäurefadens der Stärke USP 2-0 mit einer Ag°/CTAB-Beschichtung (10.000fache Vergrößerung),

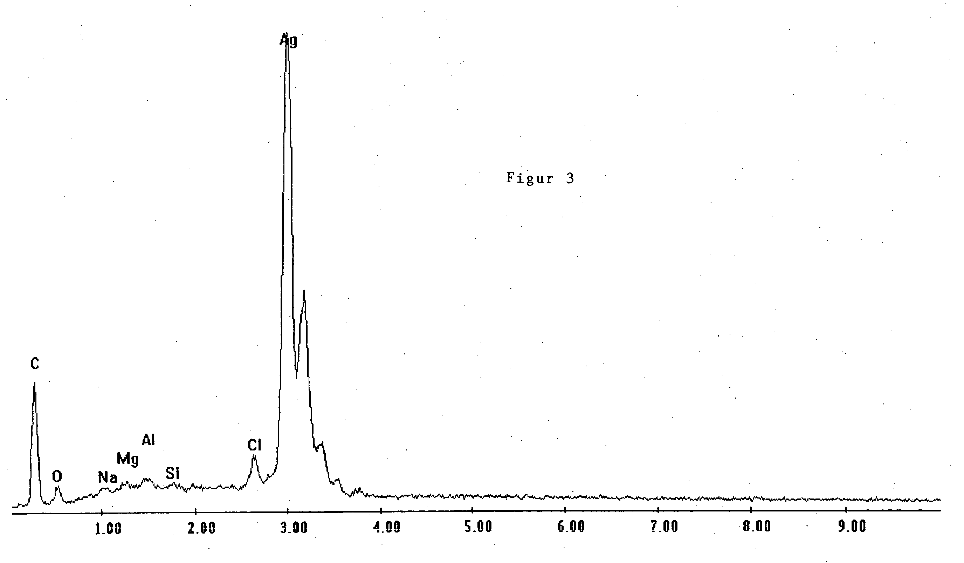

- Fig. 3:: Untersuchung eines Polyglykolsäurefadens der Stärke USP 2-0, welcher eine Ag°/CTAB-Beschichtung aufweist, mittels EDX-Analyse (Energy Dispersiv X-ray Analysis).

### Beispiel

### 1. Herstellung eines Silber Ag°/CTAB Orgänosols

Bei Raumtemperatur werden 25.0 mL einer wässrigen 0,100 molaren Silbernitratlösung mit 228 mg (0,625 mmol) Cetyltrimethylammoniumbromid (CTAB) versetzt, so dass die Lösung 0,025 molar an CTAB ist. Die wässrige Lösung wird dann mit 25.0 mL einer 0,200 molaren (189 mg, 5.00 mmol) wässrigen Natriumborhydridlösung verdünnt. Anschließend werden 50.0 mL destilliertes Dichlormethan hinzugefügt und 50 mg Natriumchlorid als Feststoff unter Rühren hinzugegeben. Dabei tritt eine starke Braunfärbung und ein Aufschäumen der Lösung ein und die Silbernanopartikel gehen in die organische Phase über, wodurch diese eine dunkelbraune Farbe annimmt. Zur besseren Durchmischung werden beide Phasen in einem Scheidetrichter mehrmals für einige Minuten geschüttelt. Als ein qualitativer Silbernachweis kann die Absorption der Silberbande bei etwa 415 nm herangezogen werden. Das auf diese Weise hergestellte Organosol weist einen Silberanteil von 629 ppm auf, wie durch ICP OES (Inductively-Coupled Plasma Optical Emission Spectrometry) Analytik festgestellt wurde.

### 2. Herstellung eines antimikrobiell ausgestatteten Nähfadens

Mit 50 mL der unter 1. hergestellten Lösung wird ein 25 Meter langes Stück eines unbeschichteten Safilfaden aus reiner Polyglykolsäure der USP(United States Pharmacopoe)-Stärke 2-0 im Tauchverfahren beschichtet. Nach Trocknen des Fadens auf einer Petrischale im Vakuumofen bei 80 °C für 4 Stunden verbleiben 645 ppm Silber auf dem chirurgischen Nähfaden wie mittels ICP OES Analytik festgestellt wurde.

Der Nähfaden wurde anschließend in 1 cm Fadenstücke geschnitten, mittels Ethylenoxid sterilisiert und mikrobiologischen Tests unterzogen.

### 3. Mikrotiterplatten-Proliferationsassay

Mit dem unter 1. hergestellten Faden wurde ein Mikrotiterplatten-Proliferationsassay durchgeführt, wobei die antimikrobielle Wirksamkeit eines Komplexmaterials aus Silbernanopartikeln und Cetyltrimethylammoniumbromid (Ag°/CTAB-Komplex) gegenüber drei verschiedenen Mikroorganismen (Staphylococcus aureus, Pseudomonas aeruginosa und Escherichia coli) untersucht wurde (Tabelle 1). Hierzu wurde die prozentuale Wachstumshemmung dieser hochresistenten Keime innerhalb der ersten sechs Stunden in Kälberserum bestimmt. Da CTAB ebenfalls eine mikrobiozide Wirkung aufweist, wurde diese Substanz als Kontrolle mitgemessen. Hierzu wurde ein 25m langer Safilfaden aus reiner Polyglykolsäure der USP-Strärke 2-0 mit ca. 50 ml einer 0,025 molaren Cetyltrimethylammoniumbromid-Dichlor-methanlösung beschichtet. Die Wachstumshemmung wurde durch die UV-Absorption einer mit einem Farbstoff versetzten und mit Kälberserum angereicherten Phosphatpufferlösung bestimmt, wobei der Farbstoff mit den Mikroorganismen reagiert.

**Tabelle 1: Ergebnisse des Proliferationsassays**

| **Erreger** | **Wachstumshemmung** | **Wachstumshemmung** |
|---|---|---|
| | **durch Ag⁰ / CTAB [%]** | **durch CTAB [%]** |
| Staphylococcus aureus (ATCC 33592) | 100 | 100 |
| Escherichia coli (ATCC 11229) | 93 | 8 |
| Pseudomonas aeruginosa (ATCC 15442) | 98 | 97 |

### 4. Agarplattendiffusionstest

Zusätzlich zu der antimikrobiellen Wirksamkeit wurde auch die Kurzzeitwirkung der Beschichtung in einem Agarplattendiffusionstest unterschucht. Hierzu wurde ein Faden der oben beschriebenen Art (Safilfaden aus reiner Polyglykolsäure der USP-Strärke 2-0) in eine phosphatgepufferte Saline gegeben und mit 5*10⁴ keimbildenden Einheiten (KBE) pro Keim inokkuliert. Anschließend wurde die Lösung auf die Platte aufgetragen. Hieraus wurde die Reduktionsrate nach 6 und 12 Stunden über eine Messung der optischen Dichte bestimmt. Die Ergebnisse sind in Tabelle 2 wiedergegeben.

**Tabelle 2: Ergebnisse des Agarplattendiffusionstests**

| **Testorganismus** | **Reduktionsfaktor nach 6 Stunden** | **Reduktionsfaktor nach 24 Stunden** |
|---|---|---|
| P. aeruginosa (ATCC 9027) | 2,2 | Keimzahl < 10 KBE/mL |
| S. aureus (ATCC 6538) | 1,0 | 1,7 |
| C. albicans (ATCC 10231) | 1,6 | 1,6 |
| S. epidermidis (ATCC12228) | 2,5 | Keimzahl < 10 KBE/mL |
| E. coli (ATCC 8739) | 1,3 | 4,2 |

Aus der Tabelle 2 geht hervor, dass die Beschichtung des Safil-Fadens mit einem Ag°/CTAB-Komplex in fast allen Fällen zu einer Verringerung der Keimpopulation führt.

### 5. Mechanische Eigenschaften eines Ag°/CTAB-beschichteten Safil-fadens

Ein Safilfaden, wie unter 1. hergestellt, wurde hinsichtlich seiner linearen Reißkraft wie seines Dehnungsverhaltens untersucht und diesbezüglich mit einem unbeschichteten Safilfaden (Safilfaden aus reiner Polyglykolsäure der USP-Strärke 2-0) verglichen (Tabelle 3).

**Tabelle 3: Untersuchung von mechanischen Eigenschaften**

| **mechanische Eigenschaften** | **unbeschichteter Faden** | **Ag⁰/CTAB** |
|---|---|---|
| lineare Reißkraft [N] | 70,51 ± 1,18 | 70,99 ± 1,17 |
| Dehnung [%] | 34,45 ± 0,76 | 39,01 ± 1,23 |

Die Tabelle 3 zeigt, dass sich die betreffenden mechanischen Eigenschaften nach der Beschichtung des Safil-Fadens im Wesentlichen nicht verändern.

## Patentansprüche

1. Medizintechnisches Produkt mit einer antimikrobiellen Ausstattung aus einem Komplexmaterial aus Metallnanopartikeln und Liganden, wobei es sich bei den Liganden zur Verhinderung einer Agglomeration der Partikel um eine oberflächenaktive Substanz handelt, **dadurch gekennzeichnet, dass** es sich bei der oberflächenaktiven Substanz um Cetyltrimethylammoniumhalogenid handelt.

2. Medizintechnisches Produkt nach Anspruch 1, **dadurch gekennzeichnet, dass** die Ausstattung auf der Oberfläche des Produktes, insbesondere in Form einer Beschichtung, vorgesehen ist.

3. Medizintechnisches Produkt nach Anspruch 1 oder 2, **dadurch gekennzeichnet, dass** die Ausstattung im Inneren des Produktes vorgesehen ist.

4. Medizintechnisches Produkt nach einem der vorhergehenden Ansprüche, **dadurch gekennzeichnet, dass** jeder Metallnanopartikel hüllenartig von mindestens einem Cetyltrimethylammoniumhalogenid umgeben ist.

5. Medizintechnisches Produkt nach einem der vorhergehenden Ansprüche, **dadurch gekennzeichnet, dass** es sich bei dem Cetyltrimethylammoniumhalogenid um Cetyltrimethylammoniumbromid handelt.

6. Medizintechnisches Produkt nach einem der vorhergehenden Ansprüche, **dadurch gekennzeichnet, dass** es sich bei den Metallnanopartikeln um Silbernanopartikel handelt.

7. Medizintechnisches Produkt nach einem der vorhergehenden Ansprüche, **dadurch gekennzeichnet, dass** die Metallnanopartikel eine zerklüftete Morphologie aufweisen.

8. Medizintechnisches Produkt nach einem der vorhergehenden Ansprüche, **dadurch gekennzeichnet, dass** die Metallnanopartikel einen Durchmesser von 80 bis 180 nm, insbesondere 100 bis 150 nm, aufweisen.

9. Medizintechnisches Produkt nach einem der vorhergehenden Ansprüche, **dadurch gekennzeichnet, dass** der Beschichtungsgehalt der Metallnanopartikel auf dem Produkt zwischen 0,05 und 3 Gew.-%, vorzugsweise 0,05 und 1 Gew.-%, bezogen auf das Gesamtgewicht des Produktes, liegt.

10. Medizintechnisches Produkt nach einem der vorhergehenden Ansprüche, **dadurch gekennzeichnet, dass** das Produkt ein von der antimikrobiellen Ausstattung verschiedenes Material, insbesondere in Form einer Beschichtung, aufweist.

11. Medizintechnisches Produkt nach Anspruch 10, **dadurch gekennzeichnet, dass** das Material bioresorbierbar ist.

12. Medizintechnisches Produkt nach Anspruch 10 oder 11, **dadurch gekennzeichnet, dass** das Material ein Polymer, insbesondere ein hydrolysierbares Polymer, vorzugsweise ein Polyester und/oder Polyurethan, ist.

13. Medizintechnisches Produkt nach Anspruch 12, **dadurch gekennzeichnet, dass** das Polymer ein Co- oder Terpolymer, insbesondere auf der Basis von Glykolsäure, ε-Caprolacton und/oder Trimethylencarbonat, ist.

14. Medizintechnisches Produkt nach einem der Ansprüche 10 bis 13, **dadurch gekennzeichnet, dass** der Gehalt des Materials 1 bis 10 Gew.-%, vorzugsweise 1 bis 6 Gew.-%, bezogen auf das Gesamtgewicht des Produktes, beträgt.

15. Medizintechnisches Produkt nach einem der vorhergehenden Ansprüche, **dadurch gekennzeichnet, dass** es sich bei dem Produkt um ein eindimensionales Textilprodukt, insbesondere um eine Faser, vorzugsweise um ein Garn, handelt.

16. Medizintechnisches Produkt nach einem der vorhergehenden Ansprüche, **dadurch gekennzeichnet, dass** es sich bei dem Produkt um ein textiles Flächengebilde, insbesondere ein Vlies, Gewebe, Gewirk, Geflecht oder Gestrick, handelt.

17. Medizintechnisches Produkt nach einem der vorhergehenden Ansprüche, **dadurch gekennzeichnet, dass** es sich bei dem Produkt um ein temporäres oder dauerhaftes Implantat für den menschlichen oder tierischen Körper handelt.

18. Medizintechnisches Produkt nach einem der Ansprüche 1 bis 16, **dadurch gekennzeichnet, dass** es sich bei dem Produkt um ein medizinisches Instrument handelt.

19. Medizintechnisches Produkt nach einem der vorhergehenden Ansprüche, **dadurch gekennzeichnet, dass** das Produkt aus einem Metall oder einer Metalllegierung, insbesondere Titan, Edelstahl, Magnesium, Tantal oder einer Legierung davon, besteht.

20. Medizintechnisches Produkt nach einem der vorhergehenden Ansprüche, **dadurch gekennzeichnet, dass** das Produkt aus einem nicht resorbierbaren oder mindestens teilweise resorbierbaren Polymer besteht.

21. Medizintechnisches Produkt nach einem der vorhergehenden Ansprüche, **dadurch gekennzeichnet, dass** das Produkt aus einem keramischen Werkstoff besteht.

22. Medizintechnisches Produkt nach einem der vorhergehenden Ansprüche, **dadurch gekennzeichnet, dass** das Produkt Poren, vorzugsweise interkonnektierende Poren, aufweist.

23. Medizintechnisches Produkt nach einem der vorhergehenden Ansprüche, **dadurch gekennzeichnet, dass** das Produkt sterilisierbar ist, insbesondere in sterilisierter Form vorliegt.

24. Verfahren zur Herstellung eines medizintechnischen Produktes nach einem der Ansprüche 1 bis 23, **dadurch gekennzeichnet, dass** ein antimikrobielles Komplexmaterial aus Metallnanopartikeln und Cetyltrimethylammoniumhalogenid, insbesondere in Form einer Lösung, von außen auf das nicht ausgestattete Produkt aufgebracht wird.

25. Verfahren zur Herstellung eines medizintechnischen Produktes nach einem der Ansprüche 1 bis 23, **dadurch gekennzeichnet, dass** ein antimikrobielles Komplexmaterial aus Metallnanopartikeln und Cetyltrimethylammoniumhalogenid, insbesondere in Form einer Lösung, während der Herstellung des Produktes zugegeben wird.

26. Verfahren nach Anspruch 24 oder 25, **dadurch gekennzeichnet, dass** zusätzlich ein resorbierbares Polymer, insbesondere in Form einer Lösung, aufgebracht oder zugegeben wird.

27. Verwendung eines Komplexmaterials aus Metallnanopartikeln und Cetyltrimethylammoniumhalogenid, wobei insbesondere jeder Metallnanopartikel hüllenartig von mindestens einer oberflächenaktiven Substanz umgeben ist, als Biozid bei einem medizintechnischen Produkt, insbesondere bei einem solchen nach einem der Ansprüche 1 bis 23.

## Claims

1. Medical product having an antimicrobial endowment comprising a complexed material comprising metal nanoparticles and ligands, wherein the ligands concern a surface-active substance to prevent any agglomeration of the particles, **characterized in that** the surface-active substance concerns cetyltrimethylammonium halide.

2. Medical product according to Claim 1, **characterized in that** the endowment is provided on the surface of the product, especially in the form of a coating.

3. Medical product according to Claim 1 or 2, **characterized in that** the endowment is provided in the interior of the product.

4. Medical product according to any preceding claim, **characterized in that** every metal nanoparticle is surrounded by at least one cetyltrimethylammonium halide in a sheath-like manner.

5. Medical product according to any preceding claim, **characterized in that** the cetyltrimethylammonium halide concerns cetyltrimethylammonium bromide.

6. Medical product according to any preceding claim, **characterized in that** the metal nanoparticles concern silver nanoparticles.

7. Medical product according to any preceding claim, **characterized in that** the metal nanoparticles have a fissured morphology.

8. Medical product according to any preceding claim, **characterized in that** the metal nanoparticles are from 80 to 180 nm and especially from 100 to 150 nm in diameter.

9. Medical product according to any preceding claim, **characterized in that** the coating content of the metal nanoparticles on the product is between 0.05% and 3% by weight, preferably 0.05% and 1% by weight, based on the overall weight of the product.

10. Medical product according to any preceding claim, **characterized in that** the product includes a material which is other than the antimicrobial endowment, especially in the form of a coating.

11. Medical product according to Claim 10, **characterized in that** the material is bioresorbable.

12. Medical product according to Claim 10 or 11, **characterized in that** the material is a polymer, especially hydrolysable polymer, preferably polyester and/or polyurethane.

13. Medical product according to Claim 12, **characterized in that** the polymer is a co- or terpolymer, especially on the basis of glycolic acid, ε-caprolactone and/or trimethylene carbonate.

14. Medical product according to any one of Claims 10 to 13, **characterized in that** the material comprises from 1% to 10% by weight and preferably from 1% to 6% by weight, based on the overall weight of the product.

15. Medical product according to any preceding claim, **characterized in that** the product concerns a one-dimensional textile product, especially a fibre, preferably a yarn.

16. Medical product according to any preceding claim, **characterized in that** the product concerns a textile sheetlike structure, especially a fibrous nonwoven web, a woven fabric, a loop-formingly knitted fabric, a braided fabric or a loop-drawingly knitted fabric.

17. Medical product according to any preceding claim, **characterized in that** the product concerns a temporary or permanent implant for the human or animal body.

18. Medical product according to any one of Claims 1 to 16, **characterized in that** the product concerns a medical instrument.

19. Medical product according to any preceding claim, **characterized in that** the product consists of a metal or a metal alloy, especially titanium, stainless steel, magnesium, tantalum or an alloy thereof.

20. Medical product according to any preceding claim, **characterized in that** the product consists of a non-resorbable or at least partly resorbable polymer.

21. Medical product according to any preceding claim, **characterized in that** the product consists of a ceramic material.

22. Medical product according to any preceding claim, **characterized in that** the product has pores, preferably interconnecting pores.

23. Medical product according to any preceding claim, **characterized in that** the product is sterilizable, in particular is sterilized.

24. Process for producing a medical product according to any one of Claims 1 to 23, **characterized in that** an antimicrobial complexed material comprising metal nanoparticles and cetyltrimethylammonium halide is applied, especially in the form of a solution, to the non-endowed product from the outside.

25. Process for producing a medical product according to any one of Claims 1 to 23, **characterized in that** an antimicrobial complexed material comprising metal nanoparticles and cetyltrimethylammonium halide is added, especially in the form of a solution, during the production of the product.

26. Process according to Claim 24 or 25, **characterized in that** a resorbable polymer is additionally applied or added, especially in the form of a solution.

27. Use of a complexed material comprising metal nanoparticles and cetyltrimethylammonium halide, wherein more particularly every metal nanoparticle is surrounded by at least one surface-active substance in a sheath-like manner, as a biocide in a medical product, especially in a medical product according to any one of Claims 1 to 23.

## Revendications

1. Produit médical garni d'une structure antimicrobienne en un matériau complexe composé de nanoparticules métalliques et de ligands, dans lequel les ligands sont une substance tensioactive destinée à empêcher une agglomération des particules, **caractérisé en ce que** la substance tensioactive est un halogénure de cétyltriméthylammonium.

2. Produit médical selon la revendication 1, **caractérisé en ce que** la structure est prévue sur la surface du produit, en particulier sous la forme d'un revêtement.

3. Produit médical selon la revendication 1 ou 2, **caractérisé en ce que** la structure est prévue à l'intérieur du produit.

4. Produit médical selon l'une quelconque des revendications précédentes, **caractérisé en ce que** chaque nanoparticule métallique est entourée à la manière d'une gaine par un halogénure de cétyltriméthylammonium.

5. Produit médical selon l'une quelconque des revendications précédentes, **caractérisé en ce que** le halogénure de cétyltriméthylammonium est le bromure de cétyltriméthylammonium.

6. Produit médical selon l'une quelconque des revendications précédentes, **caractérisé en ce que** les nanoparticules métalliques sont des nanoparticules d'argent.

7. Produit médical selon l'une quelconque des revendications précédentes, **caractérisé en ce que** les nanoparticules présentent une morphologie fissurée.

8. Produit médical selon l'une quelconque des revendications précédentes, **caractérisé en ce que** les nanoparticules métalliques présentent un diamètre de 80 à 180 nm, en particulier de 100 à 150 nm.

9. Produit médical selon l'une quelconque des revendications précédentes, **caractérisé en ce que** la proportion de revêtement des nanoparticules métalliques sur le produit se situe entre 0,05 et 3 % en poids, de préférence entre 0,05 et 1 % en poids, rapportée au poids total du produit.

10. Produit médical selon l'une quelconque des revendications précédentes, **caractérisé en ce que** le produit présente un matériau différent de la structure antimicrobienne, notamment sous la forme d'un revêtement.

11. Produit médical selon la revendication 10, **caractérisé en ce que** le matériau est biorésorbable.

12. Produit médical selon la revendication 10 ou 11, **caractérisé en ce que** le matériau est un polymère, en particulier un polymère hydrolysable, de préférence un polyester et/ou un polyuréthane.

13. Produit médical selon la revendication 12, **caractérisé en ce que** le polymère est un co- ou un terpolymère, en particulier à base d'acide glycolique, de ε-caprolactone et/ou de carbonate de triméthylène.

14. Produit médical selon l'une quelconque des revendications 10 à 13, **caractérisé en ce que** la proportion du matériau vaut 1 à 10 % en poids, de préférence 1 à 6 % en poids, rapportée au poids total du produit.

15. Produit médical selon l'une quelconque des revendications précédentes, **caractérisé en ce que** le produit est un produit textile unidimensionnel, en particulier une fibre, de préférence un fil.

16. Produit médical selon l'une quelconque des revendications précédentes, **caractérisé en ce que** le produit est un produit textile plat, en particulier un non-tissé, un tissu, un article maillé, un treillis ou un tricot.

17. Produit médical selon l'une quelconque des revendications précédentes, **caractérisé en ce que** le produit est un implant temporaire ou permanent pour le corps humain ou animal.

18. Produit médical selon l'une quelconque des revendications 1 à 16, **caractérisé en ce que** le produit est un instrument médical.

19. Produit médical selon l'une quelconque des revendications précédentes, **caractérisé en ce que** le produit se compose d'un métal ou d'un alliage métallique, en particulier de titane, d'acier allié, de magnésium, de tantale ou d'un alliage de ceux-ci.

20. Produit médical selon l'une quelconque des revendications précédentes, **caractérisé en ce que** le produit se compose d'un polymère non résorbable ou au moins partiellement résorbable.

21. Produit médical selon l'une quelconque des revendications précédentes, **caractérisé en ce que** le produit se compose d'un matériau céramique.

22. Produit médical selon l'une quelconque des revendications précédentes, **caractérisé en ce que** le produit présente des pores, de préférence des pores interconnectés.

23. Produit médical selon l'une quelconque des revendications précédentes, **caractérisé en ce que** le produit est stérilisable, se présente en particulier sous forme stérilisée.

24. Procédé de fabrication d'un produit médical selon l'une quelconque des revendications 1 à 23, **caractérisé en ce que** l'on dépose un matériau complexe antimicrobien composé de nanoparticules métalliques et d'un halogénure de cétyltriméthylammonium, en particulier sous la forme d'une solution, par l'extérieur sur le produit non garni.

25. Procédé de fabrication d'un produit médical selon l'une quelconque des revendications 1 à 23, **caractérisé en ce que** l'on ajoute un matériau complexe antimicrobien, composé de nanoparticules métalliques et d'un halogénure de cétyltriméthylammonium, en particulier sous la forme d'une solution, pendant la fabrication du produit.

26. Procédé selon la revendication 24 ou 25, **caractérisé en ce que** l'on dépose ou on ajoute en plus un polymère résorbable, en particulier sous la forme d'une solution.

27. Utilisation d'un matériau complexe composé de nanoparticules métalliques et d'un halogénure de cétyltriméthylammonium, dans lequel notamment chaque nanoparticule métallique est entourée à la manière d'une gaine par au moins une substance tensioactive, comme biocide dans un produit médical, en particulier dans un produit selon l'une quelconque des revendications 1 à 23.
